# EUROPÄISCHE PATENTSCHRIFT

(11) **EP 0 718 309 B1**
(45) Veröffentlichungstag und Bekanntmachung des Hinweises auf die Patenterteilung: **07.04.1999**
(21) Anmeldenummer: 95120097.1
(22) Anmeldetag: 19.12.1995
(51) Int. Cl.: C07K 16/18, C07K 14/78, G01N 33/53

(54) **Antigene und Antikörper zum Nachweis von Kollagen I**
Antigens and antibodies for the detection of collagen I
Antigènes et anticorps pour la détection du collagène I

(30) Priorität: 23.12.1994 DE 4446232; 01.02.1995 DE 19503146
(43) Veröffentlichungstag der Anmeldung: 26.06.1996
(73) Patentinhaber: Roche Diagnostics GmbH, 68298 Mannheim (DE)
(72) Erfinder: Naser, Werner, Dr., D-82377 Penzberg (DE); Dräger, Brigitte, Dr., D-82327 Tutzing (DE); Essig, Ulrich, Dr., D-82152 Planegg (DE); Hübner-Parajsz, Christa, Dr., D-82327 Tutzing (DE); Huber, Erasmus, Dr., D-86923 Finning (DE)

(56) Entgegenhaltungen:
- EP-A- 0 699 752
- WO-A-92/21698
- DE-A- 4 225 038
- JOURNAL OF BIOLOGICAL CHEMISTRY, Bd. 263, Nr. 26, 15.September 1988 MD US, Seiten 13414-13418, SEUNG-TAEK LEE ET AL. 'Construction of a full-length cDNA encoding human Pro-alpha2(I) collagen and its expression in Pro-alpha2(I)-deficient W8 rat cells'

## Beschreibung

Die Erfindung betrifft Antigene zur Herstellung von Antikörpern gegen N-Termini von Kollagen Iα2-Ketten, ein Verfahren zur Herstellung von solchen Antigenen, Antikörper gegen N-Termini von Kollagen Iα2-Ketten, welche durch Immunisierung mit einem erfindungsgemäßen Antigen erhältlich sind, sowie die Verwendung solcher Antikörper zum Nachweis von Kollagen.

Kollagen stellt ein wichtiges Strukturprotein im Bindegewebe von Haut, Knorpel und Knochen dar. Bekannt sind 11 Typen, die jeweils aus drei Ketten bestehen. Jeder Typ setzt sich aus 3 verschiedenen Ketten zusammen, die als α1, α2 und α3 bezeichnet werden (E. Miller et al. in Methods in Enzymology 144, Structural and Contractile Proteins, ed. L. Cunningham, Academic Press Inc. 1987, p. 3 - 41). Eine charakteristische Eigenschaft des reifen Kollagens bestimmter Gewebe, wie insbesondere Knochen oder Knorpel, ist die Quervernetzung von nebeneinander liegenden Fasern durch Hydroxylysylpyridinolin oder Lysylpyridinolin (D. Fujimoto et al., J. Biochem 83 (1978), 863 - 867; D. Eyre et al., Ann. Rev. Biochem 53 (1984), 717-748 und D. Eyre, Methods in Enzymology 144 (1987), 115-139). Diese Quervernetzungen können als biochemischer Marker für den spezifischen Nachweis von Kollagen genutzt werden (Z. Gunja-Smith et al., Biochem. J. 197 (1981), 759-762). Beim Abbau von extrazellulärem Kollagen gelangen Hydroxylysylpyridinolin- oder Lysylpyridinolinderivate, welche Peptidseitenketten enthalten oder freie Pyridinolin-Derivate mit Lysyl- oder Hydroxylysylresten, wie in der WO 91/10141 beschrieben, in Körperflüssigkeiten wie Blut oder Urin. Der Nachweis dieser Verbindungen in Körperflüssigkeiten ergibt daher Hinweise auf den Abbau von extrazellulärem Kollagen, wie er z.B. bei der Osteoporose sowie infolge von Tumoren des Knochengewebes auftritt. Zum Nachweis von solchen Hydroxylysyl- oder Lysylpyridinolinen mit Peptidseitenketten wurden in der WO89/12824 monoklonale Antikörper beschrieben, welche durch Immunisierung mit entsprechenden quervernetzten Kollagenfragmenten erhalten wurden, die aus dem Urin isoliert werden können. Auch bei den in WO 91/08478 und WO 92/21698 beschriebenen Verfahren erfolgt der Nachweis von Kollagen über einen Antikörper gegen natürliche, d.h. in vivo erzeugte quervernetzte Abbauprodukte von Kollagen.

Ein Nachteil dieser aus natürlichen Quellen isolierten Peptide ist, daß keine sichere Quelle für eine reproduzierbare Herstellung der Antigene oder Bindepartner im Test vorhanden ist. Ein weiterer Nachteil der aus natürlichen Quellen isolierten Peptide ist die Gefahr einer Kontamination mit infektiösem Material.

Definierte Antigene können z.B. durch chemische Synthese eines Peptids erhalten werden, welches einem Epitop des Antigens entspricht. Werden hierfür kleine Peptide mit einem Molekulargewicht von etwa 700 - 1500 D verwendet, so ist die Bindung an ein Trägermolekül erforderlich, um ein Antigen mit immunogener Wirkung zu erhalten. Durch die Bindung an das Trägermolekül darf dabei die Struktur des Epitops nicht verändert werden. Die Kupplung an das Trägermolekül erfolgt daher bislang bevorzugt an den Enden der Peptidkette in ausreichendem Abstand von dem vermuteten Epitopbereich (Laboratory Technics in Biochemistry and Molecular Biology, Synthetic Polypeptides as Antigens, Editors R.H. Burdon und P.H. van Knippenberg, Elsevier, Amsterdam, New York, Oxford 1988, Seiten 95-100).

Ein Problem bei der chemischen Synthese eines definierten Antigens, das einem natürlichen Abbauprodukt von quervernetztem Kollagen entspricht, stellt die aus der Quervernetzung resultierende Hydroxylysyl- oder Lysylpyridinolin-Struktur dar, deren chemische Synthese aufwendig ist.

Bisher wurde immer davon ausgegangen, daß es zum Nachweis von Kollagen oder Kollagenabbauprodukten in einer Probe notwendig sei, die Quervernetzungsstrukturen an sich oder sogenannte quervernetzte Peptide, die durch die Quervernetzung der Hydroxylysyl- oder Lysylreste zustandekommen, nachzuweisen, da diese Hydroxylysyl- oder Lysylpyridinolinstruktur charakteristisch für Kollagen ist. Beispiele für solche Nachweismethoden sind in der WO89/12824, WO91/08478, WO89/04491 und der WO91/10141 beschrieben.

In DE 42 25 038 wurde erstmals gezeigt, daß zum Nachweis von Kollagen oder Kollagenfragmenten auch lineare, nicht quervernetzte Peptidsequenzen und Antikörper eingesetzt werden können. Als Immunogene wurden kurze Peptide, die einer linearen Teilsequenz von Kollagen entsprechen, benutzt, die mindestens einen Lysinrest enthalten, das im natürlichen Kollagenmolekül als Hydroxylysin vorliegt. Über die ε-Aminogruppe dieses Lysinrestes ist das Peptid an ein Trägerprotein gebunden.

In der WO 94/03813 wurden ebenfalls synthetische lineare Peptide, die einer Sequenz des nichthelikalen C- oder N-terminalen Bereichs von Kollagen entsprechen, als Antigene zur Herstellung von Antikörpern gegen Kollagen oderKollagenfragmente beschrieben. Als synthetische lineare Peptide sind alle zusammenhängenden Aminosäuresequenzen des nichthelikalen C-oder N-terminalen Bereichs von Kollagen geeignet. Diese Bereiche sind aus Chuetal., Nature 310, 337-340 (1984), Click et al., Biochemistry 9, 4699-4706 (1970), Morgan et al., J. Biol. Chem. 245, 5042-5048 (1970) und Bernard et al., Biochemistry 22, 5213-5223 (1983) bekannt. Speziell wurden Peptide aus dem C-terminalen Bereich der α1-Kette von Kollagen offenbart. Die mit diesen Immunogenen erhältlichen Antikörper können in Immunoassays zum Nachweis von Kollagenabbauprodukten in Körperflüssigkeiten verwendet werden. Lee et al., J. Biol. Chem. 263, 13413-13418 (1988) offenbart die Konstruktion einer CDNA, die humanes Pro α2 (I) Kollagen kodiert, sowie deren Expression in Rattenzellen.

Die Aufgabe der vorliegenden Erfindung war es, diese Art der Immunoassays weiter zu verbessern und die hierzu notwendigen Einsatzstoffe wie Antikörper und Antigene weiter zu optimieren. Die Antikörper müssen sich dadurch auszeichnen, daß sie die in Körperflüssigkeiten vorkommenden Kollagenabbauprodukte d.h. Kollagenfragmente spezifisch erkennen.

Gegenstand der vorliegenden Erfindung ist daher ein Antikörper gegen Kollagenabbauprodukte aus Kollagen I in Körperflüssigkeiten, der spezifisch die Aminosäuresequenz Val-Gly-Leu-Gly innerhalb Kollagen I oder von Fragmenten daraus erkennt. Diese Aminosäuresequenz entspricht den Aminosäureresten 7 bis 10 des N-terminalen Endes der Kollagen Typ I α2-Kette. Es hat sich überraschenderweise herausgestellt, daß ein Immunoassay zum Nachweis von Kollagenabbauprodukten bei Verwendung dieser Antikörper erstens die Kollagenabbauprodukte in den Körperflüssigkeiten sehr spezifisch und sensitiv nachweisen kann und daß ein solcher Immunoassay eine sehr gute Korrelation zu einem Immunoassay aufweist, der Antikörper gegen quervernetzte Kollagenabbauprodukte verwendet (WO 89/04491).

Die erfindungsgemäßen Antikörper erkennen die Aminosäuresequenzen Val-Gly-Leu-Gly spezifisch. Hierunter ist zu verstehen, daß diese Aminosäuresequenz innerhalb der in den Körperflüssigkeiten vorkommenden Kollagenabbauprodukten erkannt wird. Die dieser Sequenz benachbarten Aminosäuren können von den Antikörpern miterkannt werden. Dies ist jedoch keine Bedingung für die erfindungsgemäßen Antikörper, da bei einigen Kollagenfragmenten diese Aminosäuren auch fehlen können. Entscheidend ist, daß die Antikörper das durch die Sequenz Val-Gly-Leu-Gly charakterisierte Epitop innerhalb eines Kollagenbruchstücks erkennen. Das insgesamt vom Antikörper gebundene Epitop kann um einige Aminosäuren länger sein, beispielsweise um 1-4 Aminosäuren. Besonders bevorzugte Antikörper erkennen die Aminosäuresequenz 6-13 des N-terminalen Endes der Kollagen Typ I α2-Kette.

Die Antikörper können durch allgemein übliche Verfahren hergestellt werden. Beispielsweise können zur Immunisierung aus Körperflüssigkeiten aufgereinigte Kollagenbruchstücke, die die N-terminalen Typ Iα2-Kette beinhalten, wobei die Sequenz Val-Gly-Leu-Gly enthalten sein muß, verwendet werden. Diese Kollagenbruchstücke können ebenfalls durch enzymatische oder chemische Spaltung von Kollagen Typ I hergestellt werden. Die Antikörper, die gegen diese mehr oder weniger stark definierten Peptide gebildet werden, müssen nach der Immunisierung durch ein geeignetes Screeningverfahren, ausgesucht oder aufgereinigt werden. Hierbei wird die Reaktion der Antikörper gegen kurze Peptide, die die Sequenz Val-Gly-Leu-Gly beinhalten, ausgetestet und genutzt.

Wesentlich einfacher und daher besonders bevorzugt ist die Herstellung der Antikörper durch Immunisierung der Tiere mit einem synthetischen Peptid oder Peptid-Derivat, das eine Aminosäuresequenz entsprechend SEQ ID NO 1 oder einen Teilsequenz daraus, die mindestens die Aminosäuresequenz Val-Gly-Leu-Gly umfaßt, beinhaltet. Bevorzugt enthält das synthetische Peptid mindestens 6 Aminosäuren. Bei den neben den Aminosäuren der Sequenz Val-Gly-Leu-Gly zusätzlich enthaltenen Aminosäuren werden bevorzugt die Aminosäuren N- oder/und C-terminal dieser Sequenz angefügt, die auch in der Kollagen Typ Iα2-Kette dieser Sequenz benachbart sind. Es können aber auch andere Aminosäuren angefügt werden. Wie allgemein üblich werden die Peptide an einen immunogenen Träger beispielsweise Keyhole Limpet Hemocyanine (KLH), Rinderserumalbumin oder Edestin zur Erhöhung der Immunantwort gekoppelt. Bei den erfindungsgemäßen Antikörpern kann es sich um polyklonale sowie um monoklonale Antikörper handeln, wobei monoklonale Antikörper bevorzugt sind.

Je nach ihrer Verwendung in verschiedenen Immunoassays zum Nachweis der Kollagenabbauprodukte können die Antikörper mit einer signalerzeugenden Substanz beziehungsweise Markierungsgruppe, zum Beispiel Fluoreszenzmarkierungsgruppen, (Elektro)chemilumineszenzmarkierungsgruppen, Digoxin, Biotin oder radioaktive Gruppen, gekoppelt sein. Im Falle von Immunoassays, bei denen eine feste Phase erforderlich ist, können die Antikörper an diese direkt oder indirekt gekoppelt sein. Die Kopplung dieser Gruppen beziehungsweise der festen Phase erfolgt nach den dem Fachmann bekannten Verfahren.

Ein weiterer Gegenstand der Erfindung sind Peptide oder Peptid-Derivate, die die Aminosäuresequenz entsprechend SEQ ID NO 1 oder eine Teilsequenz daraus, die mindestens die Aminosäuresequenz Val-Gly-Leu-Gly aufweist, beinhalten. Die Aminosäuresequenzen sind Teilbereiche aus den N-terminalen nicht-helikalen Ende der Kollagen Typ Iα2-Kette. Die Peptide oder Peptid-Derivate können als Immunogene zur Herstellung der erfindungsgemäßen Antikörper, als spezifische Bindepartner und als Standardmaterial in Immunoassays Verwendung finden. Die erfindungsgemäßen Peptide können durch bekannte Syntheseverfahren mittels chemischer Methoden erzeugt werden, oder durch Klonierung und Expression einer für diese Peptide kodierenden DNA-Sequenz in einer geeigneten Wirtszelle auf gentechnische Weise hergestellt werden.

Neben Peptiden betrifft die Erfindung ebenfalls Peptid-Derivate. Dieser Begriff umfaßt Peptide, in denen eine oder mehrere Aminosäuren durch eine chemische Reaktion derivatisiert worden sind. Beispiele sind insbesondere solche Moleküle, in denen das Backbone und/oder freie Aminosäuregruppen derivatisiert worden sind. Die erfindungsgemäßen Peptid-Derivate besitzen eine im wesentlichen äquivalente Spezifität und/oder Affinität der Bindung an die erfindungsgemäßen Antikörper.

Unter Peptid-Derivate werden auch peptidmimetische Substanzen verstanden, die eine im wesentlichen äquivalente Spezifität und/oder Affinität der Bindung an die erfindungsgemäßen Antikörper wie die zuvor genannten Peptide aufweisen. Peptidmimetische Substanzen sind Verbindungen, die Peptide in ihrer Wechselwirkung mit den Antikörpern ersetzen können und gegenüber den nativen Peptiden eine erhöhte Stabilität aufweisen können. Methoden zur Herstellung sind beschrieben bei Giannis und Kolter, Angew. Chem. 105 (1993), 1303-1326, Lee et al., Bull. Chem. Soc. Jpn. 66 (1993), 2006-2010 und Dorsch et al., Kontakte (Darmstadt) (1993) (2), 48-56.

Je nach ihrer Verwendung als Immunogen zur Herstellung der Antikörper oder als Einsatzstoff in Immunoassays, können die erfindungsgemäßen Peptide oder Peptid-Derivate an immunogene Träger, wie bereits oben beschrieben, signalerzeugende Substanzen beziehungsweise Markierungsgruppen z.B. Fluoreszenzmarkierungsgruppen, Digoxin, Biotin oder radioaktive Gruppen gekoppelt sein. Im Fall von Immunoassays, bei denen eine feste Phase erforderlich ist, können die Peptide oder Peptid-Derivate direkt oder indirekt beispielsweise über eine Streptavidin/Biotin Wechselwirkung an diese Festphase, beispielsweise Tubes, Kugeln, Latexpartikel usw., gebunden sein. Die Kopplung erfolgt nach gängigen Verfahren.

Wie bereits angesprochen, können diese erfindungsgemäßen Peptide oder Peptid-Derivate zur Herstellung von Antikörper verwendet werden.

Ein weiterer Gegenstand der Erfindung ist ein Verfahren zur Herstellung von Antikörpern gegen Kollagenabbauprodukte von Kollagen durch Immunisierung mit einem erfindungsgemäßen Peptid oder Peptidderivat, das die Aminosäuresequenz entsprechend SEQ ID NO 1 oder eine Teilsequenz daraus, die mindestens die Aminosäuresequenz Val-Gly-Leu-Gly aufweist, beinhaltet. Bevorzugt besitzt das Peptid oder Peptid-Derivat eine Länge von mindestens 6 Aminosäuren und ist an einen immunogenen Träger gekoppelt. Nach erfolgter Immunisierung werden die gewünschten Antikörper aus dem Serum der immunisierten Tiere nach bekannten Verfahren isoliert. Vorzugsweise erfolgt die Isolierung des gewünschten Antikörpers über Immunadsorption an einem an einen Träger, vorzugsweise Sepharose, gekoppeltes Peptid, das die Aminosäuresequenz entsprechend SEQ ID NO loder eine Teilsequenz daraus, die mindestens die Aminosäuresequenz Val-Gly-Leu-Gly aufweist, beinhaltet.

Ein bevorzugter Gegenstand der Erfindung ist ein Verfahren zur Herstellung monoklonaler Antikörper gegen quervernetztes Kollagen durch Immunisierung mit einem erfindungsgemäßen Peptid oder Peptid-Derivat, Immortalisierung der Milzzellen der immunisierten Tiere, Klonierung derjenigen immortalisierten Milzzellen, welche den gewünschten Antikörper produzieren und Isolierung des Antikörpers aus den klonierten Zellen oder dem Kulturüberstand dieser Zellen.

Die Immunisierung erfolgt in den hierfür üblicherweise verwendeten Tieren, vorzugsweise werden Mäuse oder Kaninchen verwendet.

Die Immortalisierung der Milzzellen der immunisierten Tiere erfolgt nach dem Fachmann geläufigen Verfahren, wie z.B. der Hybridomtechnik (Köhler und Milstein, Nature 256 (1975), 495 - 497) oder durch Transformation mit dem Epstein-Barr-Virus (EBV-Transformation). Zum Nachweis derjenigen immortalisierten Zellen, die den gewünschten Antikörper produzieren, wird eine Probe des Kulturüberstands in einem üblichen Immunoassay mit dem zur Immunisierung verwendeten erfindungsgemäßen Peptid oder Peptid-Derivat inkubiert und untersucht, ob ein Antikörper an dieses Peptid oder Peptid-Derivat bindet.

Die Konzentration von Abbauprodukten von Kollagen ist ein wichtiger diagnostischer Marker für das Ausmaß einer Osteolyse. Mit Hilfe der erfindungsgemäßen polyklonalen und monoklonalen Antikörper ist es daher möglich, über die Bestimmung der Konzentration der Abbauprodukte von natürlichem Kollagen in Körperflüssigkeiten das Ausmaß einer Osteolyse zu bestimmen.

Ein weiterer Gegenstand der Erfindung ist daher die Verwendung eines erfindungsgemäßen polyklonalen oder monoklonalen Antikörpers zur Bestimmung der Osteolyse durch Inkubation des Antikörpers mit einer Probe und Bestimmung der an den Antikörper bindenden Kollagenabbauprodukte beziehungsweise ein Immunoassay zum Nachweis von Kollagen I oder Kollagenfragmenten in einer Probe, wobei ein erfindungsgemäßer Antikörper verwendet wird.

Als Probe wird eine Körperflüssigkeit, in der sich Kollagenabbauprodukte befinden können, vorzugsweise Serum, Plasma oder Urin eingesetzt.

Prinzipiell sind zum Nachweis alle gängigen immunologischen Verfahren geeignet. Vorzugsweise erfolgt die Bestimmung der Bindung des Antikörpers an Kollagen I oder Kollagenfragmente über einen kompetitiven Test. Beim kompetitiven Test kann die Inkubation der Antikörper mit der Probe und mit dem zusätzlich enthaltenen erfindungsgemäßen Peptid oder Peptid-Derivat sowohl gleichzeitig als auch sequentiell erfolgen. Dabei kompetieren die in der Probe enthaltenen Kollagenfragmente bevorzugt mit dem erfindungsgemäßen Peptid oder Peptid-Derivat um die Bindung an den Antikörper, so daß die Bindung des Antikörpers an das erfindungsgemäße Hapten ein Maß für die Menge an Antigen in der Probe darstellt. Bei einem heterogenen kompetitiven Immunoassay, bei dem eine Trennung der flüssigen von der festen Phase erfolgt, kann sowohl der Antikörper oder das Peptid markiert beziehungsweise an die feste Phase gebunden sein. Die genaue Menge an Antigen in der Probe kann dann in üblicher Weise durch Vergleich mit einem in gleicher Weise behandelten Standard ermittelt werden.

Es können alle dem Fachmann bekannten kompetitiven Testformate zum Nachweis verwendet werden. Beispielsweise kann der Nachweis über einen turbidimetischen Inhibitions Immunoassay (TINIA) oder einen Latexpartikel Immunoassay (LPIA) erfolgen. Beim TINIA wird das erfindungsgemäße Peptid oder Peptid-Derivat an einen Träger wie beispielsweise Dextran gebunden (EP-A-0 545 350). Dieses Polyhapten konkurriert mit dem in der Probe enthaltenen Analyten um die Bindung an den Antikörper. Der gebildete Komplex kann turbidimetrisch oder nephelometrisch bestimmt werden.

Beim LPIA werden Teilchen vorzugsweise Latexpartikel mit den erfindungsgemäßen Peptiden beschichtet und mit dem erfindungsgemäßen Antikörper und der Probe vermischt. Die Agglutination wird bei Vorliegen des Analyten in der Probe erniedrigt.

Ebenso geeignet zum Nachweis sind Enzym-Immunoassays (Wisdom, G. B., Clin. Chem. 22/8 (1976), 1243 - 1255 und M. Oellerich, J. Clin. Chem. Clin. Biochem. Vol 18 (1980), 197 - 208), Fluoreszenz-Polarisations-Immunoassays (FPIA) (W. Dandliker et al., J. Exp. Med. 122 (1965), 1029), die Enzyme Multiplied Immunoassay Technik (EMIT) (Gunzer et al., Kontakte III, 1980, 3-11 und K. Rubenstein, Biochemical and Biophysical Research Communications 47 (1972), 846-851) oder die CEDIA-Technologie (Henderson et al., Clinical Chemistry 32 (1986), 1637-41).

Es hat sich teilweise als vorteilhaft erwiesen, die Probe beziehungsweise das in dieser Probe enthaltene Kollagen oder die Kollagenfragmente zu denaturieren und die Epitope so der Bindung des Antikörpers besser zugänglich zu machen. Am geeignetsten wird die Probe vor der Inkubation mit dem Antikörper mit dem Denaturierungsmittel für Proteine, die dem Fachmann bekannt sind, vorbehandelt. Um die Beeinträchtigung der immunologischen Reaktion zwischen der Probe und den spezifischen Antikörpern möglichst gering zu halten oder gänzlich zu vermeiden, wird die denaturierte Probe vor der Inkubation mit dem Antikörper bevorzugt noch verdünnt. Als Denaturierungsmittel sind alle dem Fachmann bekannten Mittel hierfür geeignet. Kaliumrhodanid (KSCN) in einer Konzentration von 2 - 6 M und Tetradecyltriäthylammoniumbromid (TTAB) in einer Konzentration von 0,5 - 2 M haben sich als besonders geeignet zur Denaturierung erwiesen.

Ein weiterer Gegenstand der Erfindung ist ein Standardmaterial zur Erstellung einer Standard- oder Eichkurve in einem Immunoassay zum Nachweis von Kollagen I oder Kollagenfragmenten, dadurch gekennzeichnet, daß ein Peptid oder Peptid-Derivat enthalten ist, das die Aminosäuresequenz entsprechend SEQ ID NO 1 oder eine Teilsequenz daraus, die mindestens die Aminosäursequenz Val-Gly-Leu-Gly aufweist, beinhaltet.

Die Erfindung wird durch die folgenden Beispiele in Verbindung mit dem Sequenzprotokoll näher erläutert.

SEQ ID NO. 1 zeigt die Sequenz eines erfindungsgemäßen Peptids, das den Aminosäuren 1-13 des N-terminalen Endes der Kollagen Typ Iα2-Kette entspricht. Xaa bedeutet Gln oder Pyr (Pyroglutamin).

### Beispiel 1

### 1.1 Peptidsynthese

Die eine Teilsequenz der Aminosäuresequenz von Kollagen entsprechend SEQ ID NO 1 aufweisenden Peptide 1 (1 Gln-Tyr-Asp-Gly-Lys-Gly-Val-Gly-Leu-Gly-Pro-Gly-13Pro)-Cys (= CollIα2(1-13, 1 Gln)-Cys) und 2 (1 Pyr-Tyr-Asp-Gly-Lys-Gly-Val-Gly-Leu-Gly-Pro-Gly-13Pro)-Cys (= CollIα2(1-13, 1 Pyr)-Cys) werden mittels Fluorenylmethyloxycarbonyl-(Fmoc)-Festphasenpeptidsynthese an einem Labortec SP 640 Peptide Synthesizer hergestellt. Dazu werden jeweils 4,0 Äquivalente von folgenden Fmoc-Aminosäurederivaten verwendet:
Tyr mit tert.-Butyl-Schutzgruppe
Lys mit tert.-Butyloxycarbonyl-Schutzgruppe
Cys mit S-tert.-Butyl-Schutzgruppe

Alle restlichen Aminosäurederivate (Gln, Pyr, Asp, Gly, Val, Leu, Pro) wurden ohne Seitenketten-Schutzgruppe eingesetzt.

Die Aminosäuren oder Aminosäurenderivate werden in N-Methylpyrrolidon gelöst. Das Peptid wird an 3 g 4-(2',4'-Dimethoxyphenyl-Fmoc-Anünomethyl)-Phenoxy-Harz (Tetrahedron Letters 28 (1987), 2107) mit einer Beladung von 0,87 mmol/g synthetisiert (JACS 95 (1973), 1328). Die Kupplungsreaktionen werden bezüglich Fmoc-Aminosäurederivat mit 4,4 Äquivalenten Dicyclohexylcarbodiimid und 4,8 Äquivalenten N-Hydroxybenzotriazol in Dimethylformamid als Reaktionsmedium während 60 Minuten durchgeführt. Der Kupplungserfolg wird mittels Kaiser-Test (Anal. Biochem. 34 (1970), 595) an dem mit Isopropanol gewaschenen Syntheseharz kontrolliert. Sofern sich hierbei ein noch nicht vollständiger Umsatz ergibt, wird durch Nachkuppeln gemäß den oben angegebenen Bedingungen, der Umsatz vervollständigt. Nach jedem Syntheseschritt wird die Fmoc-Gruppe mittels 20%igem Piperidin in Dimethylformamid in 20 Minuten abgespalten. Die Harzbeladung wird mittels UV-Absorption der freigesetzten Fulven-Gruppe nach jeder Piperidinbehandlung ermittelt. Nach der Synthese beträgt die Beladung noch 0,68 mmol/g.

Die Freisetzung des Peptids vom Syntheseharz und die Abspaltung der säurelabilen Schutzgruppen erfolgt mit 80 ml Trifluoressigsäure, 5 ml Ethandithiol, 2,5 g Phenol, 2,5 ml m-Kresol und 5 ml Wasser in 60 Minuten bei Raumtemperatur. Die Reaktionslösung wird anschließend im Vakuum eingeengt, der Rückstand in Diisopropylether aufgenommen, 1/2-2 Stunden kräftig gerührt und dann abfiltriert. Das Material wird dann mittels einer Gelpermeationsschromatographie an Sephadex G15 vorgereinigt, wobei als Elutionsmittel 0,5%ige Essigsäure verwendet wird. Das erhaltene Rohmaterial wird anschließend abfiltriert und mittels einer präparativen HPLC an Nucleosil RP18 (Säule 40 mm x 250 mm 300 Å, 5 µm) über einen Gradienten von 100 % Reagens A (Wasser, 0,1 %, Trifluoressigsäure) zu 100 % Reagens B (60 % Acetonitril, 40 % Wasser, 0,1 % Trifluoressigsäure) in 120 Minuten isoliert. Die Identität des eluierten Materials wird mittels Ionenspray Massenspektrometrie geprüft.

### 1.2 Immunogensynthese

### Aktivierung von KLH mit Maleinimido-propionsäure-N-succinimidester (=MPS)

280 mg KLH-Lyophilisat werden in 15 ml 0,1 M Kaliumphosphatpuffer pH 7,0 gelöst, eine Lösung von 7,35 mg MPS = Maleinimidopropionsäure-N-hydroxysuccinimidester in 1 ml DMSO zugeben und 16 h bei Raumtemperatur gerührt. Anschließend wird zentrifugiert und der Überstand über eine AcA 202-Säule (d = 5 cm; 1 = 25 cm; equilibriert mit 0,1 M Kaliumphosphatpuffer pH 7,0) gereinigt.

Ausbeute: 129 mg KLH-MP (4,3 x 10⁻⁵ mmol); η = 3,59 mg/ml (bestimmt mit BCA-Test; BCA-Protein Assay Reagent, Pierce)

### Umsetzung von KLH-MP mit Coll1α2(1-13, 1 Gln/Pyr)-Cys

Eine Mischung von 20,33 mg CollIα2(1-13, 1 Gln)-Cys und 20,08 mg CollIα2(1-13, 1 Pyr)-Cys werden in 1 ml H₂O gelöst und zur Lösung mit der MP-aktivierten KLH gegeben. 16 h wird bei Raumtemperatur gerührt. Anschließend wird zentrifugiert und der Überstand über eine AcA 202-Säule (d = 5 cm; 1 = 25 cm, equilibriert mit 0,1 M Kaliumphosphatpuffer pH 7,0) gereinigt.

### Aktivierung von RSA mit MPS

150 mg RSA werden in 10 ml 0,1 M Kaliumphosphatpuffer pH 7,0 gelöst, eine Lösung von 11,6 mg MPS in 1 ml DMSO zugegeben und 16 h bei Raumtemperatur gerührt. Anschließend wird die Lösung über eine AcA 202-Säule (d = 5 cm; 1 = 25 cm; equilibriert mit 0,1 M Kaliumphosphatpuffer pH 7,0) gereinigt.

Ausbeute: 121 mg RSA-MP (1,75 x 10^{.3} mmol); η = 3,78 mg/ml (bestimmt mit BCA-Test)

### Umsetzung von RSA-MP mit CollIα2(1-13, 1 Gln/Pyr)-Cys

Eine Mischung von 47,13 mg CollIα2(1-13, 1 Gln)-Cys und 46,53 mg CollIα2(1-13, 1 Pyr)-Cys wird in 3 ml H₂O gelöst und zur Lösung mit MP-aktiviertem RSA gegeben. 16 h wird bei Raumtemperatur gerührt. Anschließend wird die Lösung über eine AcA 202-Säule (d = 5 cm; 1 = 25 cm; equilibriert mit 0,1 M Kaliumphosphatpuffer pH 7,0) gereinigt.

Ausbeute: 124 mg Immunogen; η = 2,59 mg/ml (bestimmt mit BCA-Test)

### 1.3 Synthese der Peptide für die PEPSCAN-Analyse

Die Peptide wurden mittels Fmoc (Fluorenyloxycarbonyl)-Festphasensynthese hergestellt. Die Reaktionen wurden an einem multiplen Synthesizer der Firma Zinsser (SMP 360) Peptidsynthesizer durchgeführt. Die Kupplungsreaktionen wurden bezüglich Fmoc-Aminosäure-Derivat mit 1.1 Äquivalenten Dicyclohexylcarbodiimid und 1.0 Äquivalenten N-Hydroxybenzotriazol während 90 Minuten durchgeführt. Als Reaktionsmedium kam Dimethylformamid zum Einsatz. Die Fmoc-Gruppe wurde mittels 50 % Piperidin in DMF in 10 und 20 Minuten abgespalten. 10,0 Äquivalente von den folgenden Fmoc-Aminosäure-Derivaten kamen zum Einsatz: Asp (mit tert. Butyl-Ester-Schutzgruppe), Gly, Lys (mit tert. Butyloxycarbonyl-Schutzgruppe), Met, Tyr (mit tert. Butyl-Schutzgruppe).Gln, Val, Leu, Pro, Arg (mit PMC-Schutzgruppe).

Das Peptid wurde an 20 mg Rink-Harz (Polystyrol/1 % Divinylbenzol) mit einer Ladung von 0,50 mMol/g synthetisiert.

Hergestellt wurden Peptide mit jeweils 8 Aminosäure-Resten aus der Sequenz Coll I α 2 (1-21). SEQ. ID NO: 2: Gln, Tyr, Asp, Gly, Lys, Gly, Val, Gly, Leu, Gly, Pro, Met, Gly, Leu, Met, Gly, Pro, Arg, Gly.

Die Freisetzung des Peptids erfolgt mit 1 ml Trifluoressigsäure, 50 µl Ethandithiol, 50 µl Thio-Kresol, 25 µl Thioanisol und 25 pl Wasser in 3 Stunden bei Raumtemperatur. Das Peptid wurde aus der Abspaltlösung mit Diisopropylether gefällt.

Zur Biotinylierung des Peptidantigens wurde ein Moläquivalent möglichst konzentriert (Löslichkeit ist von der Aminosäuresequenz abhängig) in Argon-gesättigtem Kaliumphosphatpuffer (0,1 mol/l, pH 8,0) gelöst und mit 3 Äquivalenten D-Biotinyl-ε-aminocapronsäure-N-hydroxysuccinimidester gelöst in Argon-gesättigtem Dimethylformamid (Lösung von 1 µmol Reagenz in 5 µl DMF) versetzt.Man rührte das Reaktionsgemisch 2 Stunden bei Raumtemperatur unter Argon. Das biotinylierte Produkt wird gewonnen durch Abtrennen der niedermolekularen Bestandteile durch Gelfiltration über NAP-15 Fertigsäulen (Pharmacia) und anschließender Lyophilisation. Die Ausbeuten lagen zwischen 40 % und 90 %. Als Analytik dienten für die Reinheit HPLC, HPCE und TLC, für die Identität LSI-MS (Molpeak) und TLC mit spezifischen Anfärbereagentien (p-Dimethylaminozimtaldehyd auf Biotin) und Gehaltsbestimmung über Mikroanalyse.

### Beispiel 2

### Herstellung von monoklonalen Antikörpern

### Immunisierung von Mäusen

12 Wochen alte Balb/c-Mäuse wurden mit 100 µg Immunogen (Coll I α2 (1-13, Gln/Pyr)-Cys-KLH oder CollIα2 (1-13, Gln/Pyr)-Cys-RSA in komplettem Freundschem Adjuvans intraperitoneal erstimmunisiert.

Nach 6 Wochen folgten drei weitere Immunisierungen intraperitoneal in monatlichen Abständen. Dabei wurde jeder Maus 100 µg Immunogen in inkomplettem Freundschem Adjuvans verabreicht.

Acht Tage nach der 4. Immunisierung erfolgte eine retroorbitale Blutentnahme, um Mausserum für eine analytische Bewertung zu erhalten. Die Ergebnisse der Immunisierung mit dem Peptid, das an KLH gekoppelt war, (CollIα2 (1-13, Gln/Pyr)-Cys-KLH) waren im Vergleich zum RSA-Trägerprotein besser. Zur weiteren Untersuchung wurde aus den 15 Mäusen, die mit dem KLH-Immunogen immunisiert wurden, die 2 Mausseren 328/14 und 328/11 näher charakterisiert (vergleiche Beispiel 3.1).

Drei Tage, zwei Tage und am letzten Tag vor der Fusion folgten weitere Immunisierungen intravenös mit je 100 µg Immunogen in PBS-Puffer.

### Fusion und Klonierung

### Herstellung der Milzzellsuspension

Mäuse wurden durch Genickbruch getötet und die Milzen steril entnommen. Die Milzzellen wurden in RPMI 1640-Basismedium mittels einer Pinzette aus der Bindegewebshülle ausgedrückt. Die Zellsuspension wurde durch ein Stahlsieb gerieben und anschließend in einem Zentrifugenröhrchen in RPMI 1640-Basismedium bei 200 g zentrifugiert.

### Fusion

Milzzellen einer immunisierten Maus wurden mit P3x63 Ag8-653 Myelomzellen (ATTCC-CRL 8375) im Verhältnis 1:5 gemischt und zentrifugiert (10 Minuten, 300 g, 4°C). Die Zellen wurden noch einmal mit RPMI 1640-Basismedium gewaschen und bei 400 g in einem 50 ml Spitzröhrchen zentrifugiert Der Überstand wurde abgekippt, das Zellsediment aufgelockert, 1 ml PEG (MG 4000, Merck) dazugegeben und durchpipettiert. Nach 1 Minute im Wasserbad wurden bei Raumtemperatur 5 ml RPMI 1640-Basismedium in einem Zeitraum von 5 - 6 Minuten zugetropft, durchmischt, auf 50 ml mit Medium (RPMI 1640 + 10 % FKS) aufgefüllt und anschließend 10 Minuten bei 400 g, 4°C zentrifugiert. Die sedimentierten Zellen wurden im RPMI 1640-Medium + 10 % FKS aufgenommen und in 24-well-Zellkulturplatten mit 5 x 10⁴ Milzzelen pro well in 1 ml Selektionsmedium (100 mM Hypoxanthin, 1 µg/ml Azaserin in RPMI 1640 + 10 % FKS) ausgesät. (FKS = Fötales Kälberserum).

Nach 10 Tagen wurden diese Primärkulturen auf spezifische Antikörpersynthese getestet (vergleiche Beispiel 3.3). Primärkulturen der entsprechenden Spezifität wurden mittels FACS (Zellsorter) in 96-well-Zellkulturplatten kloniert. Als Wachstumszusatz zum Medium wurde Interleukin 6 (Boehringer Mannheim, Katalog Nr. 1271172, 100 U/ml) zugegeben. Auf diese Weise wurden 6 Hybridoma-Zellinien isoliert.

### Beispiel 3

### 3.1 Pepscan-Messungen in Mausseren

Mikrotiterplatten (Fa. Nunc) wurden mit Thermo-RSA-Streptavidin beschichtet und mit einer Lösung aus 100 ng der jeweiligen biotinylierten Peptide pro ml PBS, 0,05 % Tween 20 beladen (100 µl/well). Es handelt sich um folgende Peptide:
Coll Iα2 (1-13, 1Gln), Coll I α2 (1-13, 1Pyr), ein 1:1 Gemisch aus dieses beiden Peptiden Coll I α2 (1-13, 1Gln, Pyr), Coll Iα2 (1-8), Coll I α2 (2-9) ... bis Coll Iα2 (14-21).

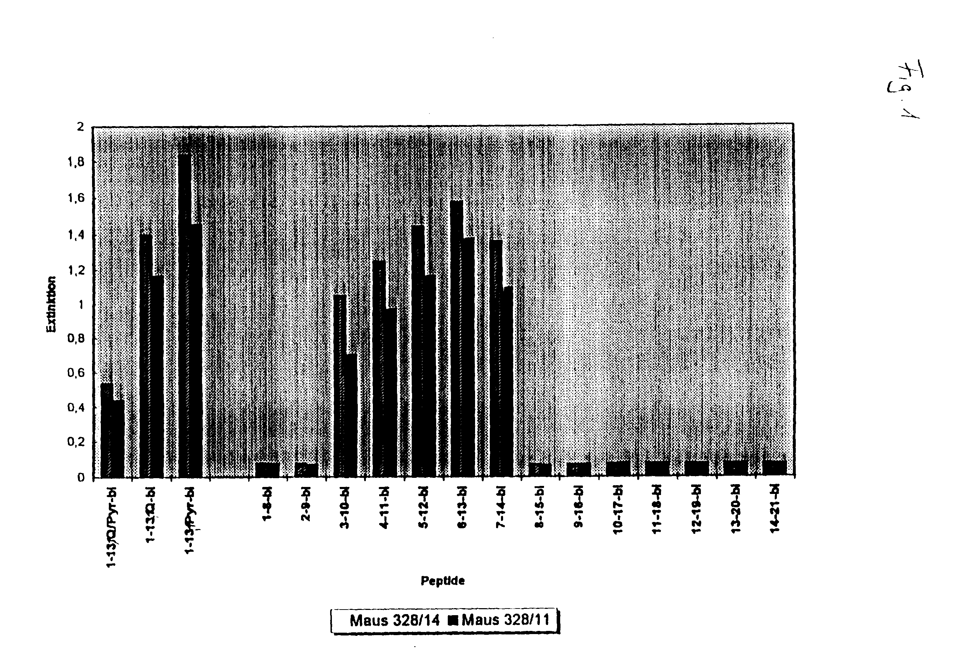

Nach einer Stunde Inkubation bei Raumtemperatur wurde zweimal mit PBS, 0,05 % Tween 20 gewaschen. Die Mausseren 328/14 beziehungsweise 328/11 wurden 1:4000 in PBS, 0,05 % Tween 20 verdünnt, 100µl dieser Lösungen wurden anschließend in die mit den Peptiden beschichteten wells pipettiert und darauffolgend 1 Stunde bei Raumtemperatur inkubiert. Danach wurde 3x mit PBS, 0,05 % Tween 20 gewaschen. Zum Nachweis der an das Peptid gebundenen Antikörper des jeweiligen Mausserums wurden 100 µl eines POD-markierten Fab-Fragments eines polyklonalen Antikörpers aus Schaf gegen Maus-Fc-y (Boehringer Mannheim, Ident-Nr. 104 75 23) in einer Konzentration von 40 mU POD/ml PBS, 0,05 % Tween zugegeben. Nach 1 Stunde Inkubation bei Raumtemperatur wurde 3x mit PBS, 0,05 % Tween 20 gewaschen. Nach Zugabe von 100 µl/well des farbbildenden Reagenzes (ABTS®, Boehringer Mannheim, Kat.-Nr. 120 45 21 und 120 45 30) wurde nach etwa 30 Minuten Inkubation bei Raumtemperatur die Extinktion bei 450/490 nm in einem Mikrotiterplattenreader der Firma SLT gemessen. Die Ergebnisse der Pepscan-Messung sind in der Fig. 1 dargestellt. Es ist deutlich zu erkennen, daß die Mausseren an die kompletten Peptide entsprechend SEQ ID NO 1 binden. Die Pepscan-Analyse mit den Octapeptiden mit Sequenzen aus dem N-terminalen Bereich der Kollagen Iα2-Kette zeigt, daß alle Antikörper die Sequenz 7-10, das heißt Val-Gly-Leu-Gly, erkennen. Diese Sequenz ist hauptsächlicher Bestandteil des für die Bindung des Antikörpers verantwortlichen Epitops.

### 3.2 EIA-Kompetitionstest

Mit den Antikörpern der Mausseren und einem Wandfixierten Peptid läßt sich ein Enzym-Immunoassay nach dem Kompetitions-Prinzip aufbauen zur Messung von Collagen-Peptiden im Urin, der eine gute Korrelation zum Osteomark®-Test der Firma Ostex aufweist.

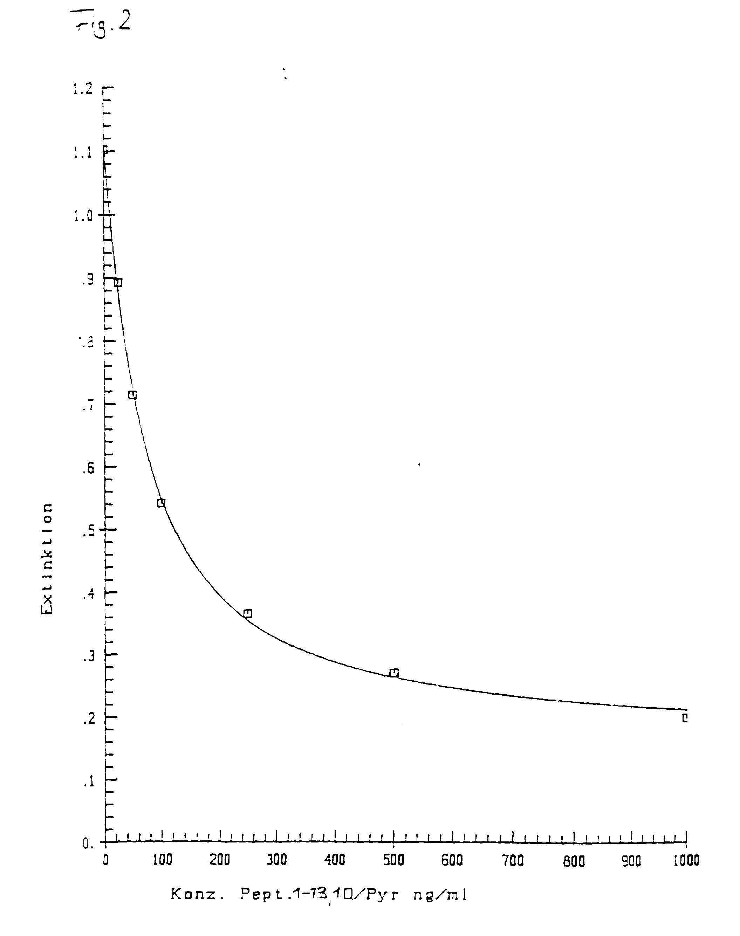

Thermo-RSA-Streptavidin beschichtete Mikrotiterplatten wurden mit dem biotinylierten Peptidgemisch CollIα2 (1-13, 1 Gln/Pyr, 1:1 Gemisch) beschichtet ( 100 ng/ml PBS, 0,05 % Tween 20, davon 100 µl/well). Nach einstündiger Inkubation bei Raumtemperatur wurde 2x mit PBS, 0,05 % Tween 20 gewaschen. Aus dem Peptidgemisch Coll I α2 (1-13, 1 Gln/Pyr) wurden Verdünnungen mit PBS, 0,05 % Tween 20 hergestellt in folgenden Konzentrationsbereichen: 0, 25, 50, 100, 250, 500 und 1000 ng/ml. Je well wurden 50 µl des jeweiligen Standards und 50 µl der Mausserumverdünnung (Mausserum 328/11, 1:4000 verdünnt in PBS, 0,05 % Tween 20) pipettiert und miteinander gemischt. Nach einstündiger Inkubation bei Raumtemperatur wurde 3x mit PBS, 0,05 % Tween 20 gewaschen und anschließend, wie im Beispiel der Pepscan-Messungen beschrieben, mit POD gelabeltem Fab-Fragment eines polyklonalen Antikörpers gegen Maus Fcγ und anschließender Farbreaktion das Meßsignal erzeugt. Die Auftragung des Meßsignals gegen die Standardkonzentration ergibt die Standardkurve (Fig. 2).

### Messung der Proben (Urin):

Statt der Standardverdünnungen wurden 50 µl Urin in den Test eingesetzt. Ansonsten erfolgte die Testführung analog der Erstellung der Standardkurve. Die Konzentrationen der Probe wurden an der Eichkurve abgelesen und anschließend auf den Creatininwert der Probe bezogen. Die Creatinin-Bestimmung erfolgte mit der enzymatischen Bestimmungsmethode Creatinin-PAP der Firma Boehringer Mannheim GmbH (Best.-Nr. 1 178 652).

Zur Vergleichsmessung wurde der Test Osteomark® der Firma Ostex verwendet; die Bezeichnung BCE bedeutet "bone collagen equivalents". Die Ergebnisse sind in der folgenden Tabelle 1 dargestellt:

**Tabelle 1**

| | Erfindungsgemäßer Test | | | Osteomark® |
|---|---|---|---|---|
| Proben | Extinktion | Konzentration ① | Konzentration ② | Konzentration ③ |
| Osteoporose | 0,932 | 16,5 | 14,42 | 432 |
| Paget | 0,661 | 64,4 | 9,36 | 282 |
| Paget | 0,813 | 34,3 | 5,31 | 116 |
| Paget | 0,742 | 46,7 | 11,9 | 362 |
| Paget | 0,822 | 32,9 | 7,5 | 306 |
| Paget | 0,672 | 61,7 | 7,5 | 221 |
| Normal | 1,023 | 8,34 | 2,89 | nicht gem. |
| Normal | 0,881 | 24,7 | 2,78 | nicht gem. |

| | | | | |
|---|---|---|---|---|
| ① Konzentration Kollagenpeptide [ng/ml] | | | | |
| ② Konzentration Kollagenpeptide [ng/ml] bezogen auf Kreatininkonzentration [µMol] | | | | |
| ③ Konzentration "bone collagen equivalents" [pMol] bezogen auf Kreatininkonzentration [nMol] | | | | |

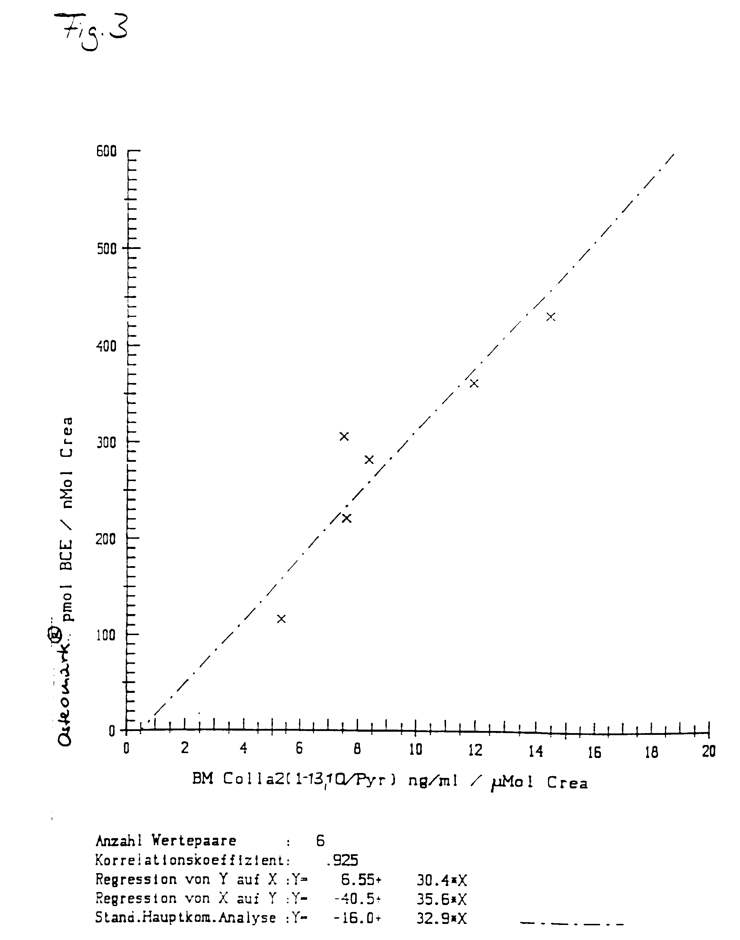

In der Fig. 3 ist die Korrelation des erfindungsgemäßen Tests zum Osteomark® Test dargestellt. Es ergab sich eine Korrelationskoeffizient von 0,925.

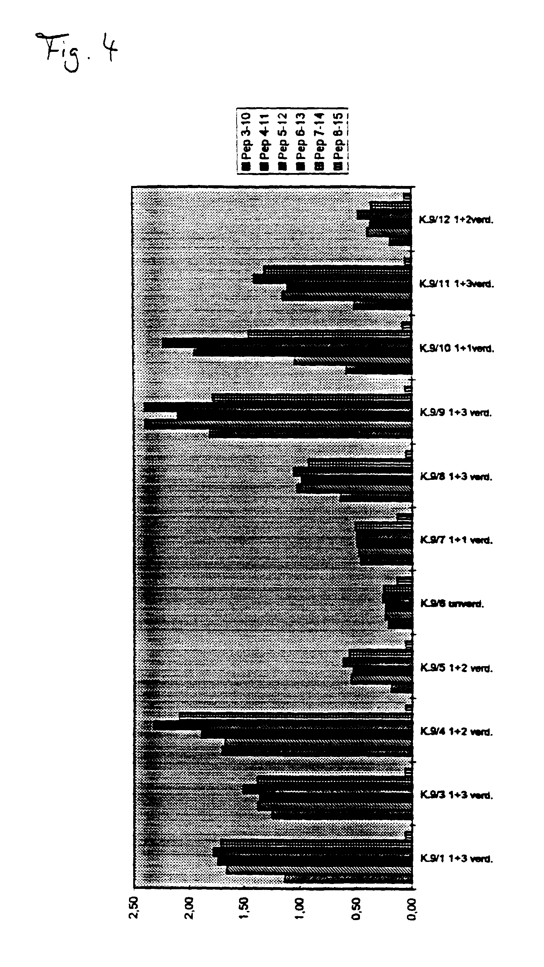

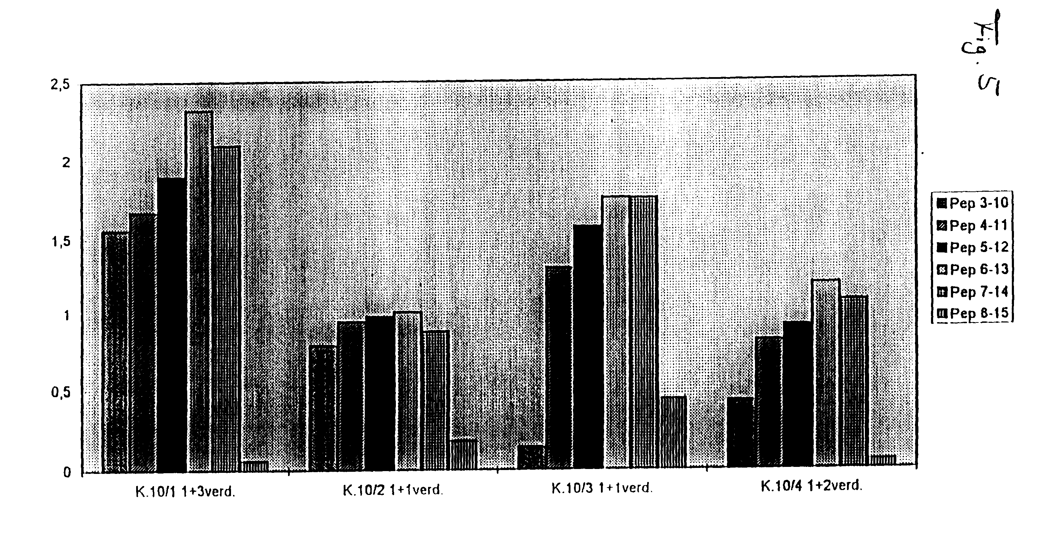

Die mit Myelomzellen fusionierten Milz-Zellen der Mäuse 328/11 und 328/14 wurden ausgesät. Mit den Kulturüberständen 9/1 bis 9/12 (Maus 328/11) und 10/1 bis 10/4 (Maus 328/14) wurde eine Pepscan-Analyse durchgeführt. Die Ergebnisse sind in Fig. 4 und 5 dargestellt.

Die Testdurchführung entspricht dem Beispiel 3.1 mit folgenden Varianten:

### 3.3 Pepscan-Messungen in Primärkulturen

Als Biotin-derivatisierte Peptide wurden nur die Peptide Coll Iα2 (3-10) bis Coll I α2 (8-15) eingesetzt. Anstelle der Mausseren wurden die Kulturüberstände 9-1 bis 9-12 beziehungsweise 10-1 bis 10-4 in unterschiedlichen Verdünnungsstufen (PBS, 0,05 % Tween 20) eingesetzt.

Der Grad der Verdünnung ist der Abbildung zu entnehmen. Unter Verwendung der Antikörperhaltigen Kulturüberstände wurden Standardkurven erstellt, Urin-Proben vermessen und Korrelationen zum Knochenresorptionsmarker Osteomark® ermittelt. Als Beispiel für das hierbei verwendete Prozedere wird ein EIA-Kompetitionstest mit den monoklonalen Antikörpern des Kulturüberstandes 9-1 (Maus 328/11) beschrieben (Beispiel 3.4).

### 3.4 EIA-Kompetitionstest mit monoklonalem Antikörper

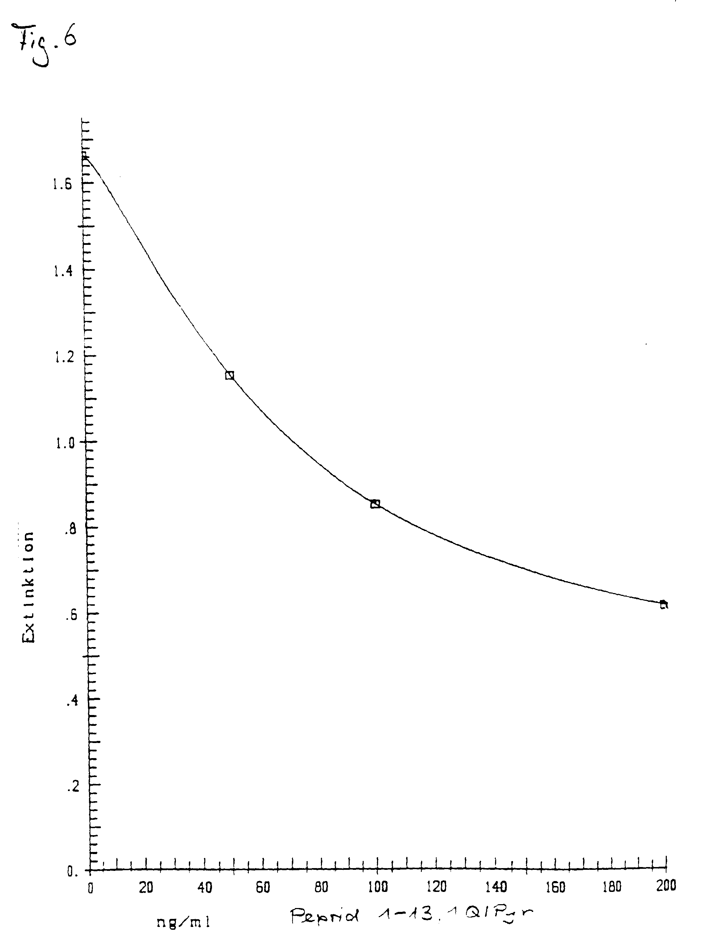

Thermo-RSA-Streptavidin beschichtete Mikrotiterplatten wurden mit dem biotinylierten Peptid Coll I α2 (6-13) beschichtet (100 ng/ml PBS, 0,05 % Tween 20, davon 100 µl/well). Nach einstündiger Inkubation bei Raumtemperatur wurde 2x mit PBS, 0,05 % Tween 20 gewaschen. Aus dem Peptidgemisch Coll I α2 (1-13, 1 Gln/Pyr) wurden durch Verdünnen mit PBS, 0,05 % Tween 20 die folgende Konzentrationen hergestellt: 0, 50, 100, 200 ng/ml. Pro well wurden 50 µl des jeweiligen Standards und 50 µl des Kulturüberstandes (1+1 verdünnt in PBS, 0,05 % Tween 20) pipettiert und gemischt. Nach einstündiger Inkubation bei Raumtemperatur wurde 3x mit PBS, 0,05 % Tween 20 gewaschen und anschließend, wie im Beispiel der Pepscan-Messungen beschrieben, mit POD gelabeltem Fab-Fragment eines polyklonalen Antikörpers gegen Maus Fcγ und anschließender Farbreaktion das Meßsignal erzeugt. Die Auftragung des Meßsignals gegen die Standardkonzentration ergibt die Standardkurve, die in Fig. 6 dargestellt ist.

### Messung der Proben (Urin):

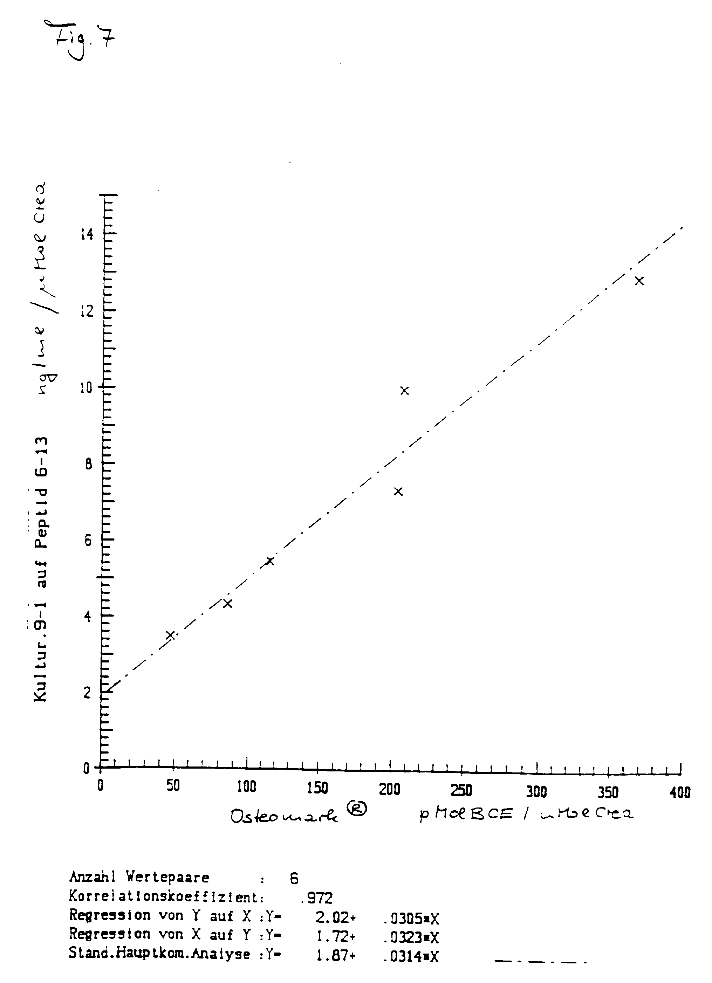

Statt der Standardverdünnungen wurden 50 µl Urin in den Test eingesetzt. Ansonsten erfolgte die Testführung analog der Erstellung der Standardkurve. Die Konzentration der Probe wurde an der Eichkurve abgelesen und anschließend auf den Creatininwert der Probe bezogen. Für die Creatinin-Bestimmung und den Test Osteomark® gilt das gleiche wie im Beispiel 3.2 erwähnt. Die Ergebnisse sind in der folgenden Tabelle 2 dargestellt. Die Korrelation zum Osteomark®-Test ist in Fig. 7 dargestellt. Es ergab sich ein Korrelationskoeffizient von 0,972.

**Tabelle 2**

| | Erfindungsgemäßer Test | | | Osteomark® |
|---|---|---|---|---|
| Proben | Extinktion | Konzentration ① | Konzentration ② | Konzentration ③ |
| Stand. | 1,663 | | | |
| Stand. 50 | 1,153 | | | |
| Stand. 100 | 0,852 | | | |
| Stand. 200 | 0,615 | | | |
| Normalurin | 1,103 | 56 | 4,34 | 86 |
| Normalurin | 1,181 | 47 | 3,48 | 48 |
| Paget | 0,866 | 96 | 12,97 | 368 |
| Paget | 1,044 | 64 | 7,36 | 204 |
| Paget | 0,735 | 135 | 5,47 | 116 |
| Osteoporose | 1,439 | 22 | 10 | 208 |

| | | | | |
|---|---|---|---|---|
| ①, ② und ③: siehe Tabelle 1 | | | | |

In der Tabelle 3 sind Daten für weitere Kulturüberstände zusammengefaßt. In allen Fällen ergab sich eine sehr gute Korrelation zum Osteomark®-Test.

**Tabelle 3**

| KÜ | Verd. | 0-Std. mE | 200 ng/ml mE | Zahl der Urine | Korrelation zu Osteomark® r |
|---|---|---|---|---|---|
| 9-1 | 1+1 | 1663 | 615 | 6 | 0.972 |
| 9-3 | 1+2 | 1391 | 397 | 5 | 0.995 |
| 9-4 | 1+2 | 1536 | 334 | 5 | 0.964 |
| 9-8 | 1+1 | 953 | 222 | 5 | 0.977 |
| 9-9 | 1+59 | 1349 | 341 | 8 | 0.925 |
| 10-2 | unverdünnt | 461 | 114 | 4 | 0.949 |
| Kü: Kulturüberstand | | | | | |
| Verd.: Verdünnung | | | | | |
| 0-Std.: niedrigster Standard | | | | | |
| 200 ng/ml: höchster Standard | | | | | |
| r: Korrelationskoeffizient | | | | | |

### SEQUENZPROTOKOLL

(iii) NUMBER OF SEQUENCES: 2

### INFORMATION FOR SEQ ID NO: 1

(i) SEQUENCE CHARACTERISTICS:
   (A) LENGTH: 13 amino acids
   (B) TYPE: amino acid
   (C) STRANDEDNESS: single
   (D) TOPOLOGY: linear
(ii) MOLECULE TYPE: peptide
(xi) SEQUENCE DESCRIPTION: SEQ ID NO: 1:

Xaa-Tyr-Asp-Gly-Lys-Gly-Val-Gly-Leu-Gly-Pro-Gly-Pro
1               5                   10

### INFORMATION FOR SEQ ID NO:2

(i) SEQUENCE CHARACTERISTICS:
   (A) LENGTH: 13 amino acids
   (B) TYPE: amino acid
   (C) STRANDEDNESS: single
   (D) TOPOLOGY: linear
(ii) MOLECULE TYPE: peptide
(xi) SEQUENCE DESCRIPTION: SEQ ID NO:2:

Gln-Tyr-Asp-Gly-Lys-Gly-Val-Gly-Leu-Gly-Pro-Met-Gly-Leu-Met-Gly-Pro-Arg-Gly
1 5 10 20

## Patentansprüche

1. Antikörper, dadurch gekennzeichnet, daß er spezifisch die Aminosäuresequenz Val-Gly-Leu-Gly innerhalb Kollagen I oder von Fragmenten daraus erkennt.

2. Antikörper gemäß Anspruch 1, erhältlich durch Immunisierung mit einem Peptid oder Peptid-Derivat, das die Aminosäuresequenz
Xaa-Tyr-Asp-Gly-Lys-Gly-Val-Gly-Leu-Gly-Pro-Gly-Pro worin
Xaa Gln oder Pyroglutamin bedeuten,
oder eine Teilsequenz daraus, die mindestens die Aminosäuresequenz
Val-Gly-Leu-Gly umfaßt, beinhaltet.

3. Antikörper gemäß Anspruch 2, dadurch gekennzeichnet, daß das Peptid oder Peptid-Derivat mindestens 6 Aminosäuren lang ist.

4. Antikörper gemäß einem der Ansprüche 1 bis 3, dadurch gekennzeichnet, daß die Antikörper monoklonal sind.

5. Verwendung eines Antikörpers gemäß einem der Ansprüche 1-3, in einem diagnostischen Verfahren zur Bestimmung der Osteolyse durch Inkubation des Antikörpers mit einer Probe und Bestimmung der an den Antikörper bindenden Kollagenabbauprodukte.

6. Immunoassay zum Nachweis von Kollagen I oder Fragmente daraus, wobei mindestens ein Antikörper gemäß einem der Ansprüche 1 - 4 verwendet wird.

7. Immunoassay gemäß Anspruch 6, nach dem Prinzip eines kompetitiven Immunoassays, wobei zusätzlich ein Peptid oder Peptid-Derivat, das die Aminosäuresequenz
Xaa-Tyr-Asp-Gly-Lys-Gly-Val-Gly-Leu-Gly-Pro-Gly-Pro worin
Xaa Gln oder Pyroglutamin bedeuten,
oder eine Teilsequenz daraus, die mindestens die Aminosäuresequenz Val-Gly-Leu-Gly umfaßt, enthalten ist.

8. Peptid oder Peptid-Derivat mit der Aminosäuresequenz Xaa-Tyr-Asp-Gly-Lys-Gly-Val-Gly-Leu-Gly-Pro-Gly-Pro worin Xaa Gln oder Pyroglutamin bedeuten, oder eine Teilsequenz daraus, die mindestens die Aminosäuresequenz Val-Gly-Leu-Gly umfaßt.

9. Peptid oder Peptid-Derivat gemäß Anspruch 8 zur Herstellung von Antikörpern, dadurch gekennzeichnet, daß es an eine immunogene Trägersubstanz gekoppelt ist.

10. Verwendung eines Peptids oder Peptid-Derivats gemäß Anspruch 8 in einem Immunoassay.

11. Verfahren zur Herstellung von Antikörpern gegen Kollagen I oder Fragmente daraus durch Immunisierung geeigneter Tiere mit einem Peptid oder Peptid-Derivat, gemäß einem der Ansprüche 8 oder 9 und Isolierung des gewünschten Antikörpers aus dem Serum des Tieres.

12. Verfahren zur Herstellung monoklonaler Antikörper gemäß Anspruch 4 durch Immunisierung eines geeigneten Tieres mit einem Peptid oder Peptid-Derivat, gemäß einem der Ansprüche 8 oder 9, Immortalisierung der Milzzellen der immunisierten Tiere, Klonierung derjenigen immortalisierten Milzzellen, die die gewünschten Antikörper produzieren und Isolierung der Antikörper aus den klonierten Zellen oder den Kulturüberständen dieser Zellen.

## Claims

1. Antibody, characterized in that it specifically recognizes the amino acid sequence Val-Gly-Leu-Gly within collagen I or fragments thereof.

2. Antibody as claimed in claim 1, obtainable by immunization with a peptide or peptide derivative containing the amino acid sequence Xaa-Tyr-Asp-Gly-Lys-Gly-Val-Gly-Leu-Gly-Pro-Gly-Pro where Xaa means Gln or pyroglutamine,
or a partial sequence thereof including at least the amino acid sequence Val-Gly-Leu-Gly.

3. Antibody as claimed in claim 2, characterized in that the peptide or peptide derivative has an amino acid length of at least 6 amino acids.

4. Antibody as claimed in one of the claims 1 to 3 characterized in that the antibodies are monoclonal.

5. Use of an antibody as claimed in one of the claims 1 to 3 in a diagnostic process for determination of osteolysis by incubation of the antibody with a sample and determination of the collagen breakdown products binding to the antibody.

6. Immunoassay for the detection of collagen I or fragments thereof where at least one antibody as claimed in one of the claims 1 to 4 is used.

7. Immunoassay as claimed in claim 6, according to the principle of the competitive immunoassay additionally containing a peptide or peptide derivative including the amino acid sequence Xaa-Tyr-Asp-Gly-Lys-Gly-Val-Gly-Leu-Gly-Pro-Gly-Pro where Xaa means Gln or pyroglutamine, or a partial sequence thereof including at least the amino acid sequence Val-Gly-Leu-Gly.

8. Peptide or peptide derivative with the amino acid sequence Xaa-Tyr-Asp-Gly-Lys-Gly-Val-Gly-Leu-Gly-Pro-Gly-Pro where Xaa means Gln or pyroglutamine, or a partial sequence thereof including at least the amino acid sequence Val-Gly-Leu-Gly.

9. Peptide or peptide derivative as claimed in claim 8 for the production of antibodies characterized in that it is coupled to an immunogenic carrier substance.

10. Use of a peptide or peptide derivative as claimed in claim 8 in an immunoassay.

11. Process for the production of antibodies against collagen I or fragments thereof by immunization of suitable animals with a peptide or peptide derivative as claimed in one of the claims 8 or 9 and isolation of the desired antibody from the animal serum.

12. Process for the production of monoclonal antibodies as claimed in claim 4 by immunization of a suitable animal with a peptide or peptide derivative as claimed in one of the claims 8 or 9, immortalization of the spleen cells of the immunized animals, cloning of the immortalized spleen cells producing the antibodies desired, and isolation of the antibodies from the cloned cells or the culture supernatants of these cells.

## Revendications

1. Anticorps caractérisé en ce qu'il détecte spécifiquement la séquence d'acides aminés Val-Gly-Leu-Gly dans le collagène I ou à partir de fragments de celui-ci.

2. Anticorps selon la revendication 1, qui peut être obtenu par l'immunisation avec un peptide ou un dérivé peptidique contenant la séquence d'acides aminés Xaa-Tyr-Asp-Gly-Lys-Gly-Val-Gly-Leu-Gly-Pro-Gly-Pro où Xaa représente Gln ou la pyroglutamine, ou une séquence partielle de celle-ci qui inclut au moins la séquence d'acides aminés Val-Gly-Leu-Gly.

3. Anticorps selon la revendication 2, caractérisé en ce que le peptide ou le dérivé peptidique contient au moins 6 acides aminés.

4. Anticorps selon l'une des revendications 1 à 3, caractérisé en ce que les anticorps sont monoclonaux.

5. Utilisation d'un anticorps selon l'une des revendications 1 à 3, dans une méthode diagnostique pour la détermination de l'ostéolyse par incubation de l'anticorps avec un échantillon et la détermination des produits de dégradation du collagène liés à l'anticorps.

6. Dosage immunologique pour la mise en évidence du collagène I ou de fragments de celui-ci où au moins un anticorps selon l'une des revendications 1 à 4 est utilisé.

7. Dosage immunologique selon la revendication 6 et suivant le principe des dosages immunologiques compétitifs et qui contient en plus un peptide ou un dérivé peptidique incluant la séquence d'acides aminés Xaa-Tyr-Asp-Gly-Lys-Gly-Val-Gly-Leu-Gly-Pro-Gly-Pro où Xaa représente Gln ou la pyroglutamine, ou une séquence partielle de celle-ci incluant au moins la séquence d'acides aminés Val-Gly-Leu-Gly.

8. Peptide ou un dérivé peptidique de la séquence d'acides aminés Xaa-Tyr-Asp-Gly-Lys-Gly-Val-Gly-Leu-Gly-Pro-Gly-Pro où Xaa représente Gln ou la pyroglutamine, ou une séquence partielle de celle-ci incluant au moins la séquence d'acides aminés Val-Gly-Leu-Gly.

9. Peptide ou un dérivé peptidique selon la revendication 8, pour la production d'anticorps, caractérisé en ce qu'il est couplé à une substance porteuse immunogéne.

10. Utilisation d'un peptide ou d'un dérivé peptidique selon la revendication 8 dans un dosage immunologique.

11. Procédé de préparation d'anticorps contre le collagène I ou de fragments de celui-ci par immunisation d'animaux appropriés avec un peptide ou un dérivé peptidique selon l'une des revendications 8 ou 9 ainsi que l'isolation de l'anticorps souhaité à partir du sérum de l'animal.

12. Méthode de production d'anticorps monoclonaux selon la revendication 4 par immunisation d'un animal approprié avec un peptide ou un dérivé peptidique selon une des revendications 8 ou 9, immortalisation des cellules de rate des animaux immunisés, clonage des cellules de rate immortalisées qui produisent les anticorps souhaités et isolation des anticorps à partir des cellules clonées ou des saillies de culture de ces cellules.
